# EUROPÄISCHE PATENTSCHRIFT

(11) **EP 0 552 429 B1**
(45) Veröffentlichungstag und Bekanntmachung des Hinweises auf die Patenterteilung: **24.04.1996**
(21) Anmeldenummer: 92118683.9
(22) Anmeldetag: 31.10.1992
(51) Int. Cl.: A61B 1/00, A61M 25/01

(54) **Endoskop mit einem steuerbaren distalen Endstück**
Endoscope with a steerable distal end portion
Endoscope avec une extrémité distale dirigeable

(30) Priorität: 18.01.1992 DE 4201280
(43) Veröffentlichungstag der Anmeldung: 28.07.1993
(73) Patentinhaber: Richard Wolf GmbH, D-75438 Knittlingen (DE)
(72) Erfinder: Heckele, Helmut, W-7134 Knittlingen (DE); Heimberger, Rudolf, W-7519 Oberdingen (DE)
(74) Vertreter: Wilcken, Thomas, Dipl.-Ing.

(56) Entgegenhaltungen:
- EP-A- 0 098 100
- EP-A- 0 183 585
- EP-A- 0 422 842
- GB-A- 2 130 885
- US-A- 4 911 148

## Beschreibung

Die Erfindung betrifft ein Endoskop mit einem steuerbaren distalen Endstück, dessen Stellung relativ zum Instrumentenschaft mit mindestens einem proximalseitig betätigbaren, durch ein beidendig festgelegtes Führungsrohr geführten Zugdraht durch Biegen veränderbar ist, wobei das Endstück an der beim Biegen auf größerem Biegeradius liegenden Seite als Rückstellfeder wirkt und an der auf kleinerem Biegeradius liegenden Seite biegeweich ausgebildet ist.

Bekannt sind Endoskope, welche bei möglichst kleinem Außendurchmesser neben Optik und Lichtleitern einen Sondenkanal besitzen und deren distale Endstücke mindestens nach einer Seite auslenkbar sind. Mit solchen dünnen Endoskopen sind Diagnose und Therapie auch in sehr schwierig zugänglichen Körperbereichen und Organen, wie z.B. im Gallengang, im Harnleiter oder in Blutgefäßen, möglich.

So ist aus der DE-B-2 402 886 ein flexibles Fiberendoskop mit ablenkbarem distalen Ende bekannt, bei dem die Abwinkelung des distalen Endes durch proximalseits betätigbare Zugdrähte erfolgt. Hierzu sind die Betätigungsdrähte durch Ringe, die das Faserbündel lose umgeben, gefädelt, und die Ringe sind in einer zur Achse parallelen Richtung mit zwei sich diametral gegenüberliegenden Schwenkfortsätzen versehen. Den Schwenkfortsätzen liegen in der entgegengesetzten Richtung entsprechende Ausschnitte gegenüber, in denen die Schwenkfortsätze des benachbarten Ringes unter Bildung jeweils eines Schwenkgelenkes gleiten. Die Schwenkfortsätze dieser Ringe sind unter entsprechender Ausbildung der Ausschnitte kreissegment-zylinderförmig gestaltet.

Aus der DE-C-2 150 595 kann man einen zum vorstehenden sehr ähnlichen Aufbau eines ablenkbaren distalen Endoskopendes entnehmen, weshalb hier nur beispielhaft auch auf diese Schrift verwiesen wird.

Auch aus der DE-B-1 766 809 ist ein Endoskop mit ablenkbarem distalen Ende bekannt, wobei das Endstück im wesentlichen aus einem schraubenförmig gewickelten Band gebildet wird, das von einer biegsamen Hülse umgeben ist. Das schraubenförmig gewickelte Band ist an einem Ende mit der Objektivfassung und an seinem anderen Ende mit einem sich proximalwärts erstreckenden flexiblen Rohr verbunden. Die Abwinkelung des distalen Endbereiches erfolgt durch Zugdrähte, die an der Objektivfassung festgelegt sind und sich proximalwärts bis zum Handhabenteil mit Zugdrahtbetätigungseinrichtung erstrecken. Die Zugdrähte sind von eng schraubenförmig gewickelten Drähten umgeben, und diese wiederum sind jeweils an ihren Enden festgelegt, also einerseits in der Steuereinrichtung und andererseits am distalen Ende des flexiblen Endoskoprohres, so daß sich der Aufbau und die Funktion eines Bowdenzuges ergeben. Zusätzlich sind bei diesen Endoskopen noch Versteifungsdrähte vorgesehen, die an der Objektivfassung und proximalwärts innerhalb des flexiblen Biegebereiches festgelegt sind, um einer Kontraktion des Biegebereiches bei Betätigung der Zugdrähte entgegenzuwirken und um ein Brechen des vorzugsweise sehr dünn ausgebildeten distalen Endbereiches, also des schraubenförmig gewickelten Bandes, möglichst zu verhindern.

Dem erstgenannten Stand der Technik haftet insbesondere der Nachteil an, daß bei einem Aufbau des ablenkbaren distalen Endbereiches aus gelenkig verbundenen Elementen, z. B. Ringen, nicht zuletzt durch die zur Herstellung der Gelenke nötigen Verbindungen, der abwinkelbare Schaft entweder einen so großen Außendurchmesser aufweist, daß ein Einsatz in dem oben angegebenen medizinischen Bereich nicht mehr möglich ist, oder aber der Außendurchmesser wird auf Kosten des verbleibenden freien Durchgangs des Endoskops gering gehalten, wodurch der Raum, z. B. für Hilfsinstrumentenkanäle, sehr stark beschränkt wird. Wählt man dagegen eine dünne Drahtspirale für den distalen Außenschaft, so muß man zur Vermeidung von Materialbrüchen schon beim bestimmungsgemäßen Biegen des ablenkbaren Endbereiches zusätzliche Versteifungsvorrichtungen, wie z. B. Versteifungsdrähte, vorsehen, welche den konstruktiven Aufbau eines entsprechenden Instrumentes unnötig komplizieren und ebenfalls wieder auf Kosten des für Hilfsinstrumente oder Spül- und Saugkanäle usw. verbleibenden freien Durchgangs gehen.

Bei mit Hilfe von Bowdenzügen ablenkbaren distalen Endoskopenden ist nachteilig, daß sich die Bowdenzugspirale unter Belastung des Zugdrahtes wellenförmig auf diesem anlegt, wodurch sowohl eine erhöhte Reibung auftritt als auch eine Verkürzung des gesamten Endoskopschaftes bewirkt wird. Dadurch wird eine optimale Abwinkelung des distalen Endstückes verhindert.

Besser sind insofern Endoskope (GB-A-21 30 885, US-A-4,911,148), bei denen das Endstück an der beim Biegen auf größerem Biegeradius liegenden Seite als Rückstellfeder wirkt und an der auf kleinerem Biegeradius liegenden Seite biegeweich ausgebildet ist. Zu diesem Zweck ist das Endstück an der auf kleinerem Biegeradius liegenden Seite durch Materialausnehmungen geschwächt, wobei diese Ausnehmungen in einem zum Endstück gehörenden biegsamen Rohr durch Schlitze gebildet sind, die parallel zueinander über einen Teil des Rohrumfanges laufen, während der von Schlitzen freie Teil des Rohres die Rückstellfeder bildet.

Bei solchen Endoskopen kommt man mit einem wenig Platz beanspruchenden Zugdraht zur Einstellung der Position des Endstückes aus, da das Endstück aufgrund seiner Rückstellfeder bei Entlastung des Zugdrahtes selbsttätig in seine gestreckte Position zurückkehren wird. Allerdings ist es bei solchen Endstücken nachteilig, daß sie wenig formstabil sind und daß deshalb die jeweils eingestellte Biegeposition häufig nicht eingehalten werden kann.

Der Erfindung liegt die Aufgabe zugrunde, die Formsteifigkeit des in Biegestellung befindlichen Endstückes und die Präzision der Ablenkung des Endstückes im Vergleich zu vorbekannten Lösungen zu verbessern.

Diese Aufgabe wird bei einem Endoskop der eingangs erwähnten Art so gelöst, daß im Endstück ein Geflechtschlauch vorgesehen ist, dessen Enden in Form von Ringkörpern versteift sind, daß der Geflechtschlauch an der auf größerem Biegeradius liegenden Seite mit einer die Ringkörper verbindenden, die Rückstellfeder bildenden Versteifung versehen ist und daß der Zugdraht am distalen Ringkörper angreift, während der proximale Ringkörper ortsfest ist.

Die Versteifungen können zweckmäßigerweise durch Verschweißen, aber auch durch Verlöten oder Verkleben von Drähten des Geflechtschlauches hergestellt werden.

Auf diese Weise lassen sich zum einen sehr stabile Ringkörper und zum anderen eine Rückstellfeder nach Art einer Blattfeder erreichen, die im Zusammenwirken mit den mit ihr verbundenen Ringkörpern exakte und gegen Verwindung sichere Biegestellungen des Endstückes gewährleistet, das außerdem einfach und wirtschaftlich hergestellt werden kann.

Durch die erfindungsgemäße Lösung ist es also möglich, den Geflechtschlauch an vorbestimmbaren Stellen verformungssteif, an anderen flexibel und an weiteren Stellen Rückstellfedern auszubilden und somit diesen Teil des Endoskops den speziellen Anforderungen angepaßt auszuführen. Im übrigen kann der Geflechtschlauch vom Endstück in den Instrumentenschaft übergehen.

Um eine präzise Übertragung der Steuerkräfte zur Auslenkung des Endstücks des Endoskops zu gewährleisten, ist das den Zugdraht führende Führungsrohr zug- und druckstabil ausgebildet, so daß es während der Betätigung des Zugdrahtes keine Längenänderung erfährt.

Die Erfindung wird nachstehend anhand der in den Zeichnungen dargestellten Ausführungsbeispiele näher erläutert. Es zeigen:
- Figur 1: einen erfindungsgemäßen Endoskopschaft im Längsschnitt,
- Figur 2: einen Schnitt entlang der Linie I-I nach Figur 1 in vergrößertem Maßstab,
- Figur 3: eine vergrößerte Darstellung des distalen Schaftendes nach Figur 1 im Längsschnitt,
- Figur 4: das distale Schaftende in der Ausführung als Geflechtschlauch,
- Figur 5: einen Längsschnitt durch das distale Schaftende entsprechend Figur 4,
- Figur 6: den proximalen Teil des Endoskopes teilweise längsgeschnitten dargestellt und
- Figur 7: einen Längsschnitt durch das distale Ende eines mit einem Ballon ausgestatteten Endoskopes.

Das erfindungsgemäße Endoskop weist im wesentlichen einen Instrumentenschaft 1 mit einem distalen Endstück 2 auf, das über einen Zugdraht 3 ablenkbar ist. Proximalwärts des distalen Endstücks 2 schließt sich ein in üblicher Weise aufgebauter und mit einem flexiblen, knicksteifen Außenmantel 8 z. B. aus Kunststoff versehener Schaftteil 4 an, der wiederum proximalseitig mit einem Handhabengehäuse 5 verbunden ist. Weiter kann der Schaft mit Lichtleitern, Bildleitern, Saug- und Spülkanälen, Instrumentenkanälen usw. in für den Fachmann geläufiger Weise ausgestattet werden.

Das ablenkbare distale Endstück 2 ist distalseitig durch eine Endoskopspitze 6 abgeschlossen, welche beispielsweise als Halter für ein nicht dargestelltes Endoskopobjektiv dienen kann und ebenfalls nicht dargestellte Durchbohrungen zur Verbindung mit den oben aufgezählten verschiedenartigen Kanälen aufweisen kann. Das ablenkbare distale Endstück 2 besteht seinerseits aus einem dünnwandigen Rohr 7 aus elastischem Material mit Federeigenschaften, welches von dem Außenmantel 8 umgeben ist.

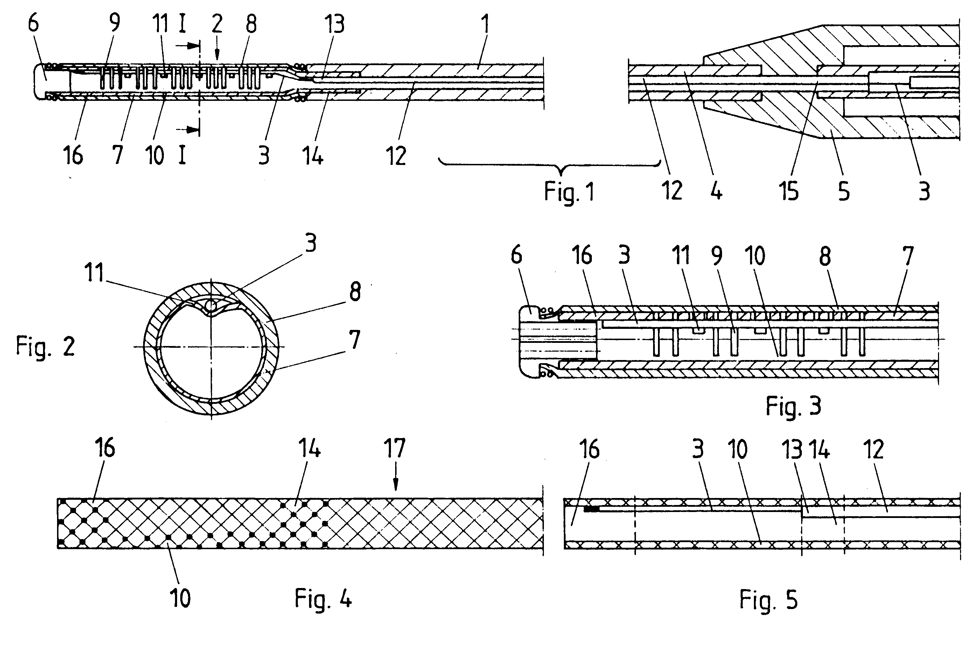

Das Rohr 7 ist, wie in Figur 1 und Figur 3 dargestellt, einseitig mit nebeneinanderliegenden, zueinander parallelen Schlitzen 9 versehen. Hierdurch wird eine nach zwei Seiten wirkende hohe Flexibilität des Rohres 7 erreicht. Der den Schlitzen 9 gegenüberliegende Teil des Rohres 7 hat keine Schlitze, wirkt praktisch als Rückstellfeder 10 und stellt damit eine Art versteifendes Gelenk dar. Der mit den Schlitzen 9 versehene Rohrabschnitt weist zusätzlich in einem gewissen Abstand zueinander zwischen den Schlitzen 9 nach innen gedrückte Vorsprünge 11 auf, in welchen der Zugdraht 3 geführt ist. Das Endstück 2 hat am distalen Ende einen verformungssteifen Ringabschnitt 16 und am proximalen Ende einen verformungssteifen Ringabschnitt 14, die axial durch das geschlitzte Rohrteil 7 und die Rückstellfeder 10 miteinander verbunden sind.

Im gesamten Verlauf des Schaftteiles 4 ist ein dünnwandiges Führungsrohr 12 vorgesehen, welches als Hülle für den Zugdraht 3 dient. Das distale Ende 13 des Führungsrohres 12 ist direkt mit dem proximal angeordneten Ringabschnitt 14 des Endstücks 2 beispielsweise durch Löten, Kleben oder andere gebräuchliche Befestigungsverfahren verbunden.

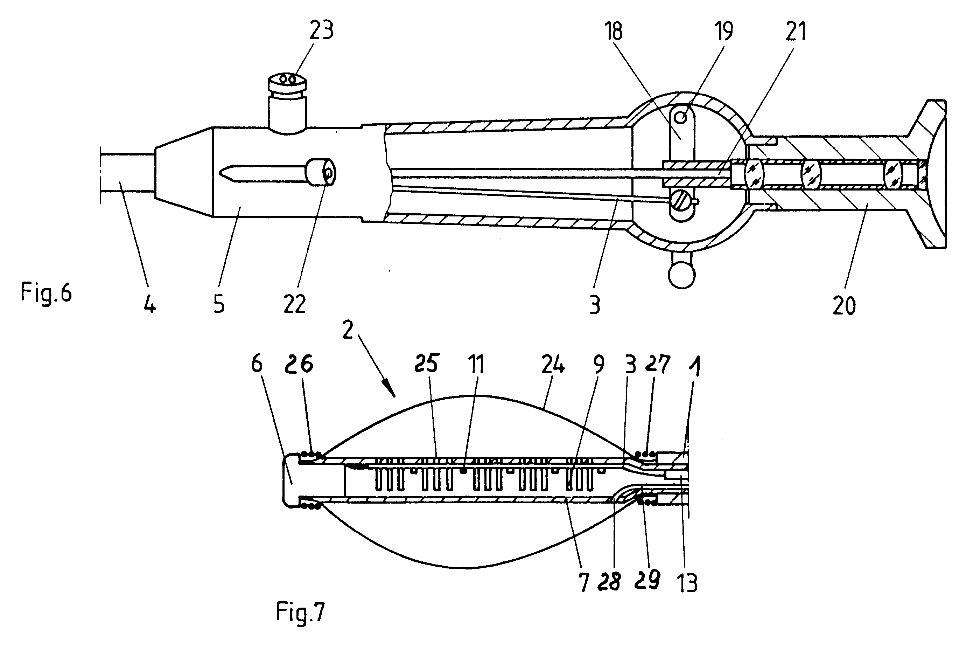

Das proximale Ende 15 des Führungsrohres 12 ist im Handhabengehäuse 5 ebenfalls fest mit diesem verbunden. Der Zugdraht 3 wird im Handhabengehäuse 5 über das proximale Ende 15 des Führungsrohres 12 hinaus bis zu einer Zugdrahtbetätigungseinrichtung geführt. Wie in Figur 6 erkennbar, umfaßt diese einen aus dem Handhabengehäuse 5 herausgeführten einseitigen Hebel 18, der um eine Achse 19 schwenkbar und an dem der Zugdraht 3 mit seinem proximalen Ende befestigt ist. Das Handhabengehäuse 5 ist mit einem Okular 20 versehen, in das das proximale Ende eines Bildleiters 21 mündet, und weist Anschlüsse 22 und 23 für einen Sondenkanal sowie für Lichtleiter auf.

Bei der Ausführung des Endstückes 2 entsprechend Figur 4 und 5 ist ein zum Endstück gehörender Abschnitt als Geflechtschlauch 17 ausgebildet, welcher beidendig die Ringabschnitte 14, 16 darstellende Versteifungszonen aufweist. Zwischen den Ringabschnitten 14, 16 ist der Geflechtschlauch 17 biegsam, jedoch an der auf seinem größeren Biegeradius liegenden Längsseite zwecks Bildung der Rückstellfeder 10 versteift. Die zu den Ringabschnitten 14, 16 sowie zur Rückstellfeder 10 gebildeten Versteifungen werden dadurch erreicht, daß die Drähte des Geflechtschlauchs 17 in den entsprechenden Bereichen miteinander verschweißt, verlötet oder verklebt werden.

Durch Betätigen des Zugdrahts 3 über die dargestellte Betätigungseinrichtung am Handhabengehäuse 5 kann das ablenkbare distale Endstück 2 des Instrumentenschaftes 1 abgewinkelt und ebenfalls durch den Zugdraht 3 wieder in die gerade oder eine schwächer abgewinkelte Position gebracht werden. Während der Abwinkelung bzw. beim Geraderichten wird der Zugdraht 3 entweder auf Zug oder auf Druck belastet. Bei beiden Belastungszuständen erweisen sich die Vorteile der Verwendung des Rohres 12 anstelle einer Bowdenzugspirale als Hülle für den Zugdraht 3, indem nämlich in beiden Fällen keine Längenänderungen des Instrumentenschaftes 1 auftreten und die Reibung zwischen Zugdraht 3 und Führungsrohr 12 sich beim Manipulieren des Abwinkelbereiches nicht erhöht, so daß somit die volle Kraft mit minimalen Verlusten zur Verfügung steht. Während der Manipulation wirkt die Rückstellfeder 10 als versteifendes Gelenk und bewirkt beim Nachlassen der Zugkraft am Zugdraht 3, daß das Endstück 2 wieder seine Ausgangsposition einnimmt.

Gemäß Figur 7 kann am distalen Ende des Endoskopes ein Ballon zum Dilatieren von Körperhöhlen vorgesehen werden. Dieser besteht aus einem äußeren flexiblen und dehnbaren Schlauch 24 und einem inneren Schlauch 25, der die Schlitze 9 abdeckt. Die sich überdeckenden Enden der beiden Schläuche sind z.B. mit Fäden 26, 27 dicht auf den Teilen 6, 7 abgebunden. In den zwischen den Schläuchen gebildeten Raum ragt die Mündung 28 eines Kanales 29, der proximalseitig an eine Druckquelle angeschlossen werden kann, um den Ballon beispielsweise mit Luft aufzublasen, wobei sich wie dargestellt der äußere Schlauch 24 vom inneren Schlauch 25 abhebt. Nach Ablassen der Luft über den Kanal 29 wird sich der äußere Schlauch wieder eng an den inneren anlegen.

Durch Verwendung von Formgedächtnismetall für den abwinkelbaren Teil des Endstückes 2 kann eine anatomisch günstige Krümmung des Endstückes vorgegeben und fixiert werden, so daß das Endstück aus einer gekrümmten Ausgangslage heraus mit dem Zugdraht, ggf. auch mit mehreren Zugdrähten, manipuliert werden kann.

## Patentansprüche

1. Endoskop mit einem steuerbaren distalen Endstück (2), dessen Stellung relativ zum Instrumentenschaft (1) mit mindestens einem proximalseitig betätigbaren, durch ein beidendig festgelegtes Führungsrohr (12) geführten Zugdraht (3) durch Biegen veränderbar ist, wobei das Endstück (2) an der beim Biegen auf größerem Biegeradius liegenden Seite als Rückstellfeder (10) wirkt und an der auf kleinerem Biegeradius liegenden Seite biegeweich ausgebildet ist, dadurch gekennzeichnet, daß im Endstück (2) ein Geflechtschlauch (17) vorgesehen ist, dessen Enden in Form von Ringkörpern (14 u. 16) versteift sind, daß der Geflechtschlauch (17) an der auf größerem Biegeradius liegenden Seite mit einer die Ringkörper (14 bzw. 16) verbindenden, die Rückstellfeder (10) bildenden Versteifung versehen ist und daß der Zugdraht (3) am distalen Ringkörper (16) angreift, während der proximale Ringkörper (14) ortsfest ist.

2. Endoskop nach Anspruch 1, dadurch gekennzeichnet, daß die Versteifungen durch Verschweißen von Drähten des Geflechtschlauches (17) hergestellt sind.

3. Endoskop nach einem der Ansprüche 1 und 2, dadurch gekennzeichnet, daß der Geflechtschlauch (17) vom Endstück (2) in den Instrumentenschaft (1) übergeht.

4. Endoskop nach einem der Ansprüche 1 bis 3, dadurch gekennzeichnet, daß das Führungsrohr (12) zug- und druckstabil ist.

5. Endoskop nach einem der Ansprüche 1 bis 4, dadurch gekennzeichnet, daß an seinem distalen Ende im Bereich des Endstückes (2) ein aufblasbarer Ballon (24, 25) vorgesehen ist.

## Claims

1. An endoscope with a steerable distal end-piece (2), the position of which relative to the instrument shank (1) being adjustable by bending with at least one pull wire (3) which is operable from the proximal side and guided through a guiding tube (12) which is fixed at both ends, said end-piece (2) on bending, acting as a restoring spring (10) on the side on the larger bending radius and, on the side on the smaller bending radius is formed supplely, characterized in that a braided sleeving (17) is provided in the end-piece (2), the ends of which are stiffened in the form of annular bodies (14 and 16); that the braided sleeving (17), on the side on the larger bending radius, is provided with a stiffening which connects the annular bodies (14 and 16) and forms the restoring spring (10) and that the pull wire (3) engages on the distal annular body (16), whilst the proximal annular body (14) is stationary.

2. An endoscope according to claim 1, characterized in that the stiffenings are produced by bonding the wires of the braided sleeving (17).

3. An endoscope according to one of claims 1 or 2, characterized in that the braided sleeving (17) runs into the instrument shank (1) from the end-piece (2).

4. An endoscope according to one of claims 1 to 3, characterized in that the guiding tube (12) has tensile and pressure stability.

5. An endoscope according to one of claims 1 to 4, characterized in that an inflatable balloon (24,25) is provided at its distal end in the region of the end-piece (2).

## Revendications

1. Endoscope ayant un bout distal (2) dirigeable dont la position par rapport à la tige (1) de l'endoscope est variable par flexion au moyen d'un fil transmetteur (3) dispose à l'intérieur d'un tuyau de guidage (12), fixé par ses deux extrémités, et manipulable du côté proximal, le bout (2) produisant un effet de ressort de rappel (10) du côté du rayon de courbure supérieur et étant flexible du côté du rayon de courbure inférieur, caractérisé en ce qu'à l'intérieur du bout (2) est prévue une gaine tressée (17) dont les extrémités sont rigidifiées aux moyens de corps annulaires (14 et 16), en ce que la gaine tressée (17) présente du côté du rayon de courbure supérieur un renforcement formant le ressort de rappel (10) et reliant entre eux les corps annulaires (14 et 16), et en ce que le fil transmetteur (3) est fixé au corps annulaire distal (16) alors que le corps annulaire proximal (14) est fixe.

2. Endoscope selon la revendication 1, caractérisé en ce que les renforcements sont obtenus par soudure de fils de la gaine tressée (17).

3. Endoscope selon l'une des revendications 1 et 2, caractérisé en ce que la gaine tressée (17) se prolonge depuis le bout (2) jusqu'à la tige (1) de l'endoscope.

4. Endoscope selon l'une des revendications 1 à 3, caractérisé en ce que le tuyau de guidage (12) est résistant à la traction et à la compression.

5. Endoscope selon l'une des revendications 1 à 4, caractérisé en ce qu'il comporte, à son extrémité distale, un ballon gonflable (24, 25) dans la région du bout distal (2).
